# EUROPEAN PATENT SPECIFICATION

(11) **EP 2 538 854 B1**
(45) Date of publication and mention of the grant of the patent: **22.08.2018**
(21) Application number: 11747977.4
(22) Date of filing: 23.02.2011
(51) Int. Cl.: A61B 17/15, A61B 17/00, A61B 17/56, A61B 34/10

(54) **CUSTOMIZED PATIENT-SPECIFIC TIBIAL CUTTING BLOCKS**
ANGEPASSTE PATIENTENSPEZIFISCHE SCHIENBEINSCHNEIDEBLÖCKE
BLOCS DE COUPE TIBIALE SPÉCIFIQUES À UN PATIENT PERSONNALISÉS

(30) Priority: 25.02.2010 US 308136 P
(43) Date of publication of application: 02.01.2013
(73) Proprietor: DePuy Products, Inc., Warsaw, IN 46581 (US)
(72) Inventor: ARAM, Luke, J., Warsaw, IN 46580 (US); BUGBEE, William, Warsaw, IN 46581 (US); ENGH, Andrew, Warsaw, IN 46581 (US); MOSKAL, Joseph, Warsaw, IN 46581 (US); PAGNANO, Mark, Warsaw, IN 46581 (US); SWANK, Michael, Warsaw, IN 46581 (US); ROSE, Bryan, South Whitley, IN 46787 (US)
(74) Representative: Curran, Clair
(86) International application number: PCT/US2011/025887
(87) International publication number: WO 2011/106395

(56) References cited:
- WO-A2-2009/076296
- WO-A2-2009/111512
- US-A1- 2009 087 276
- US-A1- 2009 088 753
- US-A1- 2009 088 761

## Description

### TECHNICAL FIELD

The present invention relates to a customized patient-specific tibial cutting block as defined in claim 1.

### BACKGROUND

Joint arthroplasty is a well-known surgical procedure by which a diseased and/or damaged natural joint is replaced by a prosthetic joint. A typical knee prosthesis includes a tibial tray, a femoral component, a polymer insert or bearing positioned between the tibial tray and the femoral component, and, in some cases, a polymer patella button. To facilitate the replacement of the natural joint with the knee prosthesis, orthopaedic surgeons use a variety of orthopaedic surgical instruments such as, for example, cutting blocks, drill guides, milling guides, and other surgical instruments. Typically, the orthopaedic surgical instruments are generic with respect to the patient such that the same orthopaedic surgical instrument may be used on a number of different patients during similar orthopaedic surgical procedures.

US-A-2009/0087276 is the closest prior art to the subject-matter of the invention and shows a tibial cutting block according to the preamble of claim 1. It discloses a customized patient-specific cutting block comprising a body having a bone-facing surface that has a customized patient-specific negative contour configured to receive (i) a portion of an anterior side of a patient's tibia that has a corresponding positive contour, and (ii) a portion of a medial side of the patient's tibia that has a corresponding positive contour such that, when viewed superiorly, an angle greater than zero is defined between a vertically-extending, bisecting plane of the body and a bisecting saggital plane of the patient's tibia when the portions of the patient's tibia are received in the customized patient-specific negative contour of the body.

### SUMMARY

According to one aspect there is provided a customized patient-specific tibial cutting block as defined in claim 1.

The customized patient-specific tibial cutting block may include a first tab extending posteriorly from the body and a second tab extending posteriorly from the body. Each of the first tab and the second tab has a bone-facing surface having a customized patient-specific negative contour configured to receive a respective portion of the proximal end of the patient's tibia that has a corresponding positive contour. The first tab and the second tab may define an opening therebetween.

The cutting guide has a cutting slot defined therein and is formed from a material different from the body. The cutting guide may be formed from a metallic material and is overmolded to the body of the customized patient-specific femoral cutting block.

The longitudinal axis of the cutting slot may define a substantially perpendicular angle with the bisecting saggital plane of the patient's tibia when the portions of the patient's tibia are received in the customized patient-specific negative contour of the body.

The angle defined between the vertically-extending, bisecting plane of the body and the bisecting sagittal plane of the patient's tibia may be between ten degrees and thirty degrees. In one example, the angle defined between the vertically-extending, bisecting plane of the body and the bisecting sagittal plane of the patient's tibia is about twenty degrees.

### BRIEF DESCRIPTION OF THE DRAWINGS

The detailed description particularly refers to the following figures, in which:
FIG. 1 is a simplified flow diagram of an algorithm for designing and fabricating a customized patient-specific orthopaedic surgical instrument;
FIG. 2 is a simplified flow diagram of a method for generating a model of a patient-specific orthopaedic instrument;
FIG. 3 is a simplified flow diagram of a method for scaling a reference contour;
FIGS. 4-6 are three-dimensional models of a patient's tibia;
FIG. 7-9 are three-dimensional models of a patient's femur;
FIG. 10 is an anterior elevation of a customized patient-specific orthopaedic surgical instrument;
FIG. 11 is a top plan view of the customized patient-specific orthopaedic surgical instrument of FIG. 10;
FIG. 12 is side elevation view of the customized patient-specific orthopaedic surgical instrument of FIG. 10;
FIG. 13 shows the customized patient-specific orthopaedic surgical instrument of FIG. 10 secured to the tibia of a patient, as viewed superiorly; and
FIG. 14 is a view similar to FIG. 13, shows an embodiment of the customized patient-specific orthopaedic surgical instrument.

### DETAILED DESCRIPTION OF THE DRAWINGS

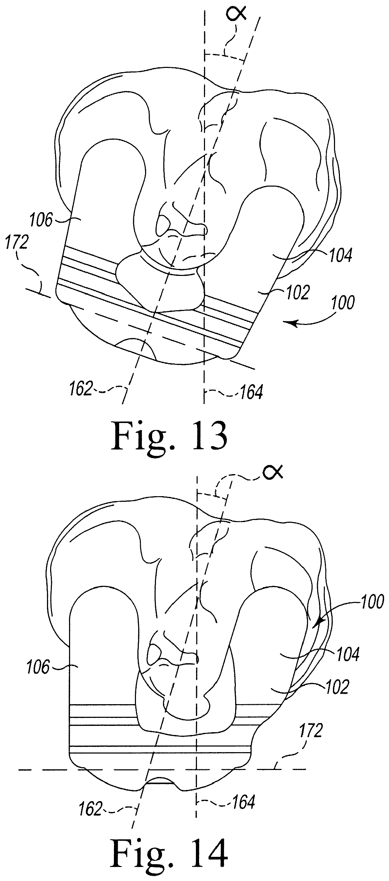

While the concepts of the present disclosure are susceptible to various modifications and alternative forms, the tibial cutting block of the invention is shown only in figure 14. It should be understood, however, that there is no intent to limit the concepts of the present disclosure to the particular forms disclosed, but on the contrary, the intention is to cover all modifications falling within the scope of the invention as defined by the appended claims.

Terms representing anatomical references, such as anterior, posterior, medial, lateral, superior, inferior, etcetera, may be used throughout this disclosure in reference to the orthopaedic implants and instruments described herein, along with a patient's natural anatomy. Such terms have well-understood meanings in both the study of anatomy and the field of orthopaedics. Use of such anatomical reference terms in the specification and claims is intended to be consistent with their well-understood meanings unless noted otherwise.

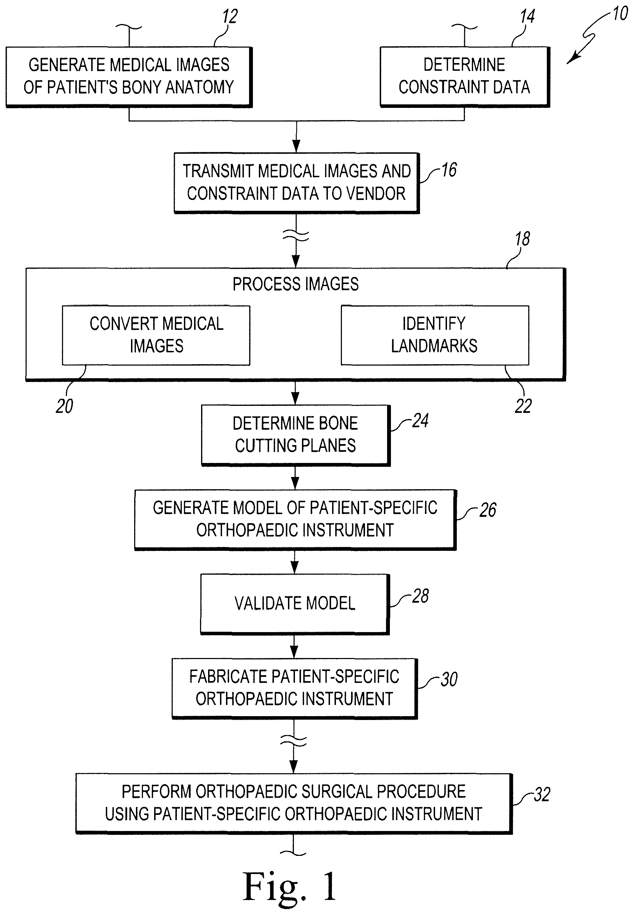

Referring to FIG. 1, an algorithm 10 for fabricating a customized patient-specific orthopaedic surgical instrument is illustrated. What is meant herein by the term "customized patient-specific orthopaedic surgical instrument" is a surgical tool for use by a surgeon in performing an orthopaedic surgical procedure that is intended, and configured, for use on a particular patient. As such, it should be appreciated that, as used herein, the term "customized patient-specific orthopaedic surgical instrument" is distinct from standard, non-patient specific orthopaedic surgical instruments that are intended for use on a variety of different patients. Additionally, it should be appreciated that, as used herein, the term "customized patient-specific orthopaedic surgical instrument" is distinct from orthopaedic prostheses, whether patient-specific or generic, which are surgically implanted in the body of the patient. Rather, customized patient-specific orthopaedic surgical instruments are used by an orthopaedic surgeon to assist in the implantation of orthopaedic prostheses.

As shown in FIG. 1, the algorithm 10 includes process steps 12 and 14, in which an orthopaedic surgeon performs pre-operative planning of the orthopaedic surgical procedure to be performed on a patient. The process steps 12 and 14 may be performed in any order or contemporaneously with each other. In process step 12, a number of medical images of the relevant bony anatomy or joint of the patient are generated. To do so, the orthopaedic surgeon or other healthcare provider may operate an imaging system to generate the medical images. The medical images may be any number and type of medical images capable of being used to generate a three-dimensional rendered model of the patient's bony anatomy or relevant joint. For example, the medical images may be any number of computed tomography (CT) images, magnetic resonance imaging (MRI) images, or other three-dimensional medical images. Additionally or alternatively, as discussed in more detail below in regard to process step 18, the medical images may a number of X-ray images or other two-dimensional images from which a three-dimensional rendered model of the patient's relevant bony anatomy may be generated. Additionally, the medical image may be enhanced with a contrast agent designed to highlight the cartilage surface of the patient's knee joint.

In process step 14, the orthopaedic surgeon may determine any additional pre-operative constraint data. The constraint data may be based on the orthopaedic surgeon's preferences, preferences of the patient, anatomical aspects of the patient, guidelines established by the healthcare facility, or the like. For example, the constraint data may include the orthopaedic surgeon's preference for a metal-on-metal interface, amount of inclination for implantation, the thickness of the bone to resect, size range of the orthopaedic implant, and/or the like. The orthopaedic surgeon's preferences may be saved as a surgeon's profile, which may used as a default constraint values for further surgical plans.

In process step 16, the medical images and the constraint data, if any, are transmitted or otherwise provided to an orthopaedic surgical instrument vendor or manufacturer. The medical images and the constraint data may be transmitted to the vendor via electronic means such as a network or the like. After the vendor has received the medical images and the constraint data, the vendor processes the images in step 18. The orthopaedic surgical instrument vendor or manufacturer process the medical images to facilitate the determination of the bone cutting planes, implant sizing, and fabrication of the customized patient-specific orthopaedic surgical instrument as discussed in more detail below. For example, in process step 20 the vendor may convert or otherwise generate three-dimensional images from the medical images. For example, in processes wherein the medical images are a number of two-dimensional images, the vendor may use a suitable computer algorithm to generate one or more three-dimensional images form the number of two-dimensional images. Additionally, in some processes, the medical images may be generated based on an established standard such as the Digital Imaging and Communications in Medicine (DICOM) standard. In such processes, an edge-detection, thresholding, watershead, or shape-matching algorithm may be used to convert or reconstruct images to a format acceptable in a computer aided design application or other image processing application. Further, in some processes, an algorithm may be used to account for tissue such as cartilage not discernable in the generated medical images. In such processes any three-dimensional model of the patient-specific instrument (see, e.g., process step 26 below) may be modified according to such algorithm to increase the fit and function of the instrument.

In process step 22, the vendor may process the medical images, and/or the converted/reconstructed images from process step 20, to determine a number of aspects related to the bony anatomy of the patient such as the anatomical axis of the patient's bones, the mechanical axis of the patient's bone, other axes and various landmarks, and/or other aspects of the patient's bony anatomy. To do so, the vendor may use any suitable algorithm to process the images.

In process step 24, the cutting planes of the patient's bone are determined. The planned cutting planes are determined based on the type, size, and position of the orthopaedic prosthesis to be used during the orthopaedic surgical procedure, on the process images such as specific landmarks identified in the images, and on the constraint data supplied by the orthopaedic surgeon in process steps 14 and 16. The type and/or size of the orthopaedic prosthesis may be determined based on the patient's anatomy and the constraint data. For example, the constraint data may dictate the type, make, model, size, or other characteristic of the orthopaedic prosthesis. The selection of the orthopaedic prosthesis may also be modified based on the medical images such that an orthopaedic prosthesis that is usable with the bony anatomy of the patient and that matches the constraint data or preferences of the orthopaedic surgeon is selected.

In addition to the type and size of the orthopaedic prosthesis, the planned location and position of the orthopaedic prosthesis relative to the patient's bony anatomy is determined. To do so, a digital template of the selected orthopaedic prosthesis may be overlaid onto one or more of the processed medical images. The vendor may use any suitable algorithm to determine a recommended location and orientation of the orthopaedic prosthesis (i.e., the digital template) with respect to the patient's bone based on the processed medical images (e.g., landmarks of the patient's bone defined in the images) and/or the constraint data. Additionally, any one or more other aspects of the patient's bony anatomy may be used to determine the proper positioning of the digital template. The digital template along with surgical alignment parameters may be presented to the orthopaedic surgeon for approval. The approval document may include the implant's rotation with respect to bony landmarks such as the femoral epicondyle, posterior condyles, sulcus groove (Whiteside's line), and the mechanical axis as defined by the hip, knee, and/or ankle centers.

The planned cutting planes for the patient's bone(s) may then be determined based on the determined size, location, and orientation of the orthopaedic prosthesis. In addition, other aspects of the patient's bony anatomy, as determined in process step 22, may be used to determine or adjust the planned cutting planes. For example, the determined mechanical axis, landmarks, and/or other determined aspects of the relevant bones of the patient may be used to determine the planned cutting planes.

In process step 26, a model of the customized patient-specific orthopaedic surgical instrument is generated. In some processes, the model is embodied as a three-dimensional rendering of the customized patient-specific orthopaedic surgical instrument. In other processes, the model may be a mock-up or fast prototype of the customized patient-specific orthopaedic surgical instrument. The particular type of orthopaedic surgical instrument to be modeled and fabricated may be determined based on the orthopaedic surgical procedure to be performed, the constraint data, and/or the type of orthopaedic prosthesis to be implanted in the patient. As such, the customized patient-specific orthopaedic surgical instrument may be any type of orthopaedic surgical instrument for use in the performance of an orthopaedic surgical procedure. For example, the orthopaedic surgical instrument may be a bone-cutting block, a drilling guide, a milling guide, and/or any other type of orthopaedic surgical tool or instrument.

The particular shape of the customized patient-specific orthopaedic surgical instrument is determined based on the planned location of the orthopaedic surgical instrument relative to the patient's bony anatomy. The location of the customized patient-specific orthopaedic surgical instrument with respect to the patient's bony anatomy is determined based on the type and determined location of the orthopaedic prosthesis to be used during the orthopaedic surgical procedure. That is, the planned location of the customized patient-specific orthopaedic surgical instrument relative to the patient's bony anatomy may be selected based on, in part, the planned cutting planes of the patient's bone(s) as determined in step 24. For example, where the customized patient-specific orthopaedic surgical instrument is a bone-cutting block, the location of the orthopaedic surgical instrument is selected such that the cutting guide of the bone-cutting block matches one or more of the planned cutting planes determined in process step 24. Additionally, the planned location of the orthopaedic surgical instrument may be based on the identified landmarks of the patient's bone identified in process step 22. The particular shape or configuration of the customized patient-specific orthopaedic surgical instrument may be determined based on the planned location of the instrument relative to the patient's bony anatomy. That is, the customized patient-specific orthopaedic surgical instrument may include a bone-contacting surface having a negative contour that matches the contour of a portion of the bony anatomy of the patient such that the orthopaedic surgical instrument may be coupled to the bony anatomy of the patient in a unique location, which corresponds to the pre-planned location for the instrument. When the orthopaedic surgical instrument is coupled to the patient's bony anatomy in the unique location, one or more guides (e.g., cutting or drilling guide) of the orthopaedic surgical instrument may be aligned to one or more of the bone cutting plane(s) as discussed above.

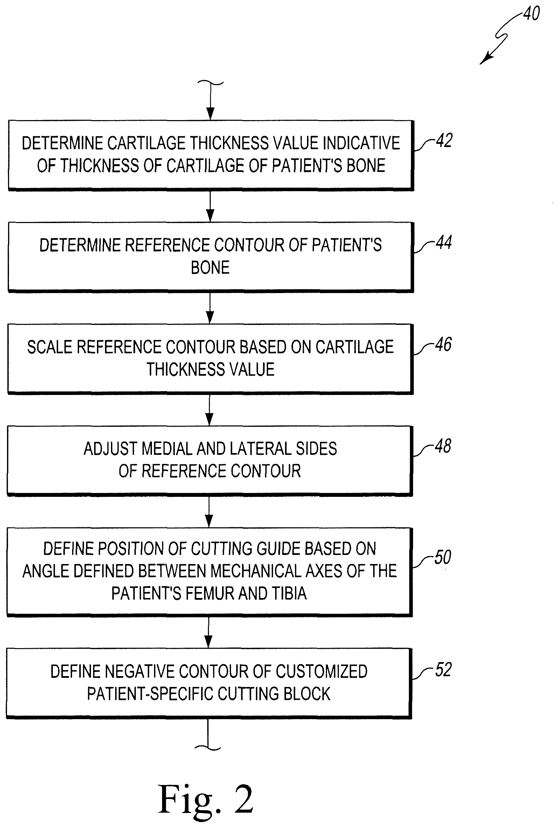

One illustrative method 40 for generating a model, such as a computer model, of a patient-specific orthopaedic instrument is illustrated in FIGS. 2 through 9. The method 40 begins with a step 42 in which a cartilage thickness value is determined. The cartilage thickness value is indicative of the average thickness of the cartilage of the patient's bone. As such, in one process, the cartilage thickness value is equal to the average thickness of cartilage for an individual having similar characteristics as the patient. For example, the cartilage thickness value may be equal to the average thickness value of individuals of the same gender as the patient, the same age as the patient, having the same activity level of the patient, and/or the like. In other processes, the cartilage thickness value is determined based on one or more medical images of the patient's bone, such as those images transmitted in process step 16.

In step 44, a reference contour of the patient's relevant bone is determined. The reference contour is based on the surface contour of a three-dimensional model of the patient's relevant bone, such as the three-dimensional model generated in step 20. Initially the reference contour is identical to a region (i.e. the region of interest such as the distal end of the patient's femur or the proximal end of the patient's tibia) of the patient's bone. That is, in some processes, the reference contour is juxtaposed on the surface contour of the region of the patient's bone.

Subsequently, in step 46, the reference contour is scaled to compensate for the cartilage thickness value determined in step 42. To do so, in one process, the scale of the reference contour is increased based on the cartilage thickness value. For example, the scale of the reference contour may be increased by an amount equal to or determined from the cartilage thickness value. However, in other processes, the reference contour may be scaled using other techniques designed to scale the reference contour to a size at which the reference contour is compensated for the thickness of the cartilage on the patient's bone.

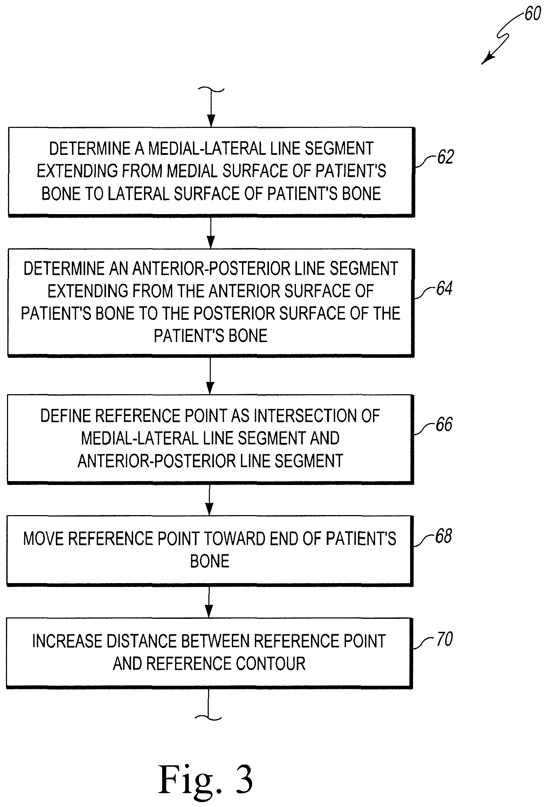

For example, in one particular process, the reference contour is scaled by increasing the distance between a fixed reference point and a point lying on, and defining in part, the reference contour. To do so, a method 60 for scaling a reference contour as illustrated in FIG. 3 may be used. The method 60 begins with step 62 in which a medial/lateral line segment is established on the three-dimensional model of the patient's relevant bone. The medial/lateral line segment is defined or otherwise selected so as to extend from a point lying on the medial surface of the patient's bone to a point lying on lateral surface of the patient's bone. The medial surface point and the lateral surface point may be selected so as to define the substantially maximum local medial/lateral width of the patient's bone.

In step 64, an anterior/posterior line segment is established on the three-dimensional model of the patient's relevant bone. The anterior/posterior line segment is defined or otherwise selected so as to extend from a point lying on the anterior surface of the patient's bone to a point lying on posterior surface of the patient's bone. The anterior surface point and the posterior surface point may be selected so as to define the substantially maximum local anterior/posterior width of the patient's bone.

The reference point from which the reference contour will be scaled is defined in step 66 as the intersection point of the medial/lateral line segment and anterior/posterior line segment. As such, it should be appreciated that the medial surface point, the lateral surface point, the anterior surface point, and the posterior surface point lie on the same plane. After the reference point is initially established in step 66, the reference point is moved or otherwise translated toward an end of the patient's bone. For example, where the patient's bone is a femur, the reference point is moved inferiorly toward the distal end of the patient's femur. Conversely, when the patient's bone is embodied as a tibia, the reference point is moved superiorly toward the proximal end of the patient's tibia. In one example, the reference point is moved a distance equal to about half the length of the anterior/posterior line segment as determined in step 64. However, in other examples, the reference point may be moved other distances sufficient to compensate the reference contour for thickness of the cartilage present on the patient's bone.

Once the location of the reference point has been determined in step 68, the distance between the reference point and each point lying on, and defining in part, the reference contour is increased in step 70. To do so, in one particular example, each point of the reference contour is moved a distance away from the reference point based on a percentage value of the original distance defined between the reference point and the particular point on the reference contour. For example, in one process, each point lying on, and defining in part, the reference contour is moved away from the reference point in by a distance equal to a percentage value of the original distance between the reference point and the particular point. In one example, the percentage value is in the range of about 5 percent to about thirty percent. In one particular example, the percentage value is about ten percent.

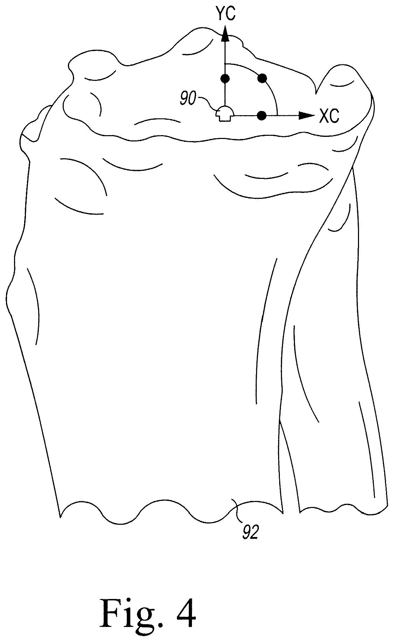

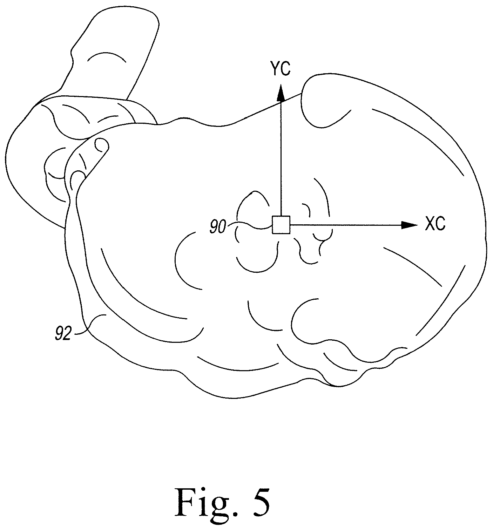

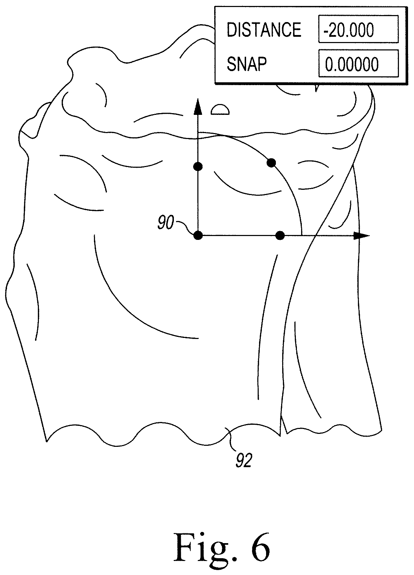

Referring now to FIGS. 4-9, in another process, the reference contour is scaled by manually selecting a local "high" point on the surface contour of the three-dimensional image of the patient's bone. For example, where the relevant patient's bone is a tibia as illustrated in FIGS. 4-6, the reference point 90 is initially located on the tibial plateau high point of the tibial model 92. Either side of the tibial plateau may be used. Once the reference point 90 is initially established on the tibial plateau high point, the reference point 90 is translated to the approximate center of the plateau as illustrated in FIG. 5 such that the Z-axis defining the reference point is parallel to the mechanical axis of the tibial model 92. Subsequently, as illustrated in FIG. 6, the reference point is moved in the distal direction by a predetermined amount. In one particular process, the reference point is moved is the distal direction by about 20 millimeters, but other distances may be used. For example, the distance over which the reference point is moved may be based on the cartilage thickness value.

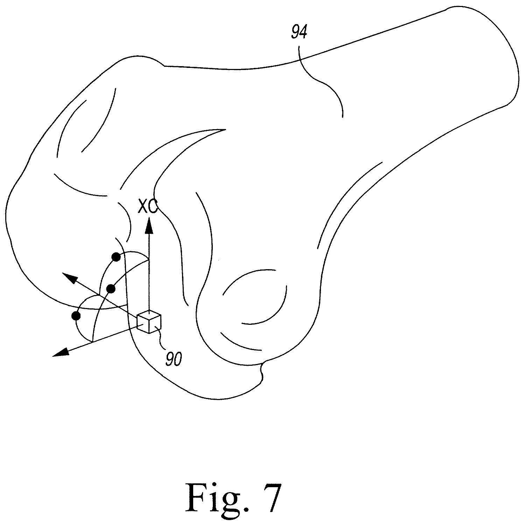

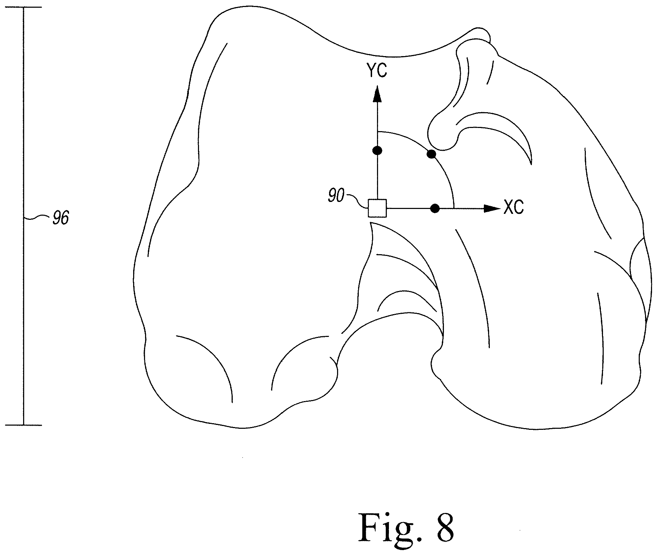

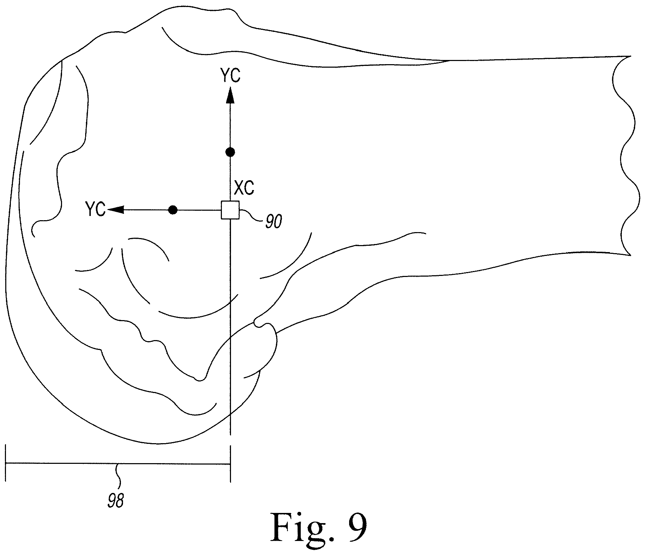

Conversely, where the relevant patient's bone is a femur as illustrated in FIGS. 7-9, the reference point 90 is initially located on the most distal point of the distal end of the femoral model 94. Either condyle of the femoral model 94 may be used. Once the reference point 90 is initially established on the most distal point, the reference point 90 is translated to the approximate center of the distal end of the femoral model 94 as illustrated in FIG. 8 such that the Z-axis defining the reference point 90 is parallel to the mechanical axis of the femoral model 92. The anterior-posterior width 96 of the distal end of the femoral model 94 is also determined. Subsequently, as illustrated in FIG. 9, the reference point is moved or otherwise translated in the proximal or superior direction by a distance 98. In one particular process, the reference point is moved in the distal or superior direction by a distance 98 equal to about half the distance 96. As such, it should be appreciated that one of a number of different techniques may be used to define the location of the reference point based on, for example, the type of bone.

Referring now back to FIG. 2, once the reference contour has been scaled in step 46, the medial/lateral sides of the reference contour are adjusted in step 48. To do so, in one process, the distance between the reference point and each point lying on, and defining in part, the medial side and lateral side of the reference contour is decreased. For example, the distance between the reference point and the points on the medial and lateral sides of the scaled reference contour are decreased to the original distance between such points. As such, it should be appreciated that the reference contour is offset or otherwise enlarged with respect to the anterior side of the patient's bone and substantially matches or is otherwise not scaled with respect to the medial and lateral sides of the patient's bone.

The reference contour may also be adjusted in step 48 for areas of the patient's bone having a reduced thickness of cartilage. Such areas of reduced cartilage thickness may be determined based on the existence of bone-on-bone contact as identified in a medical image, simulation, or the like. Additionally, information indicative of such areas may be provided by the orthopaedic surgeon based on his/her expertise. If one or more areas of reduced cartilage thickness are identified, the reference contour corresponding to such areas of the patient's bone is reduced (i.e., scaled back or down).

Additionally, one or more osteophytes on the patient's bone may be identified; and the reference contour may be compensated for such presence of the osteophytes. By compensating for such osteophytes, the reference contour more closely matches the surface contour of the patient's bone. Further, a distal end (where the patient's bone is a tibia) or a proximal end (where the patient's bone is a femur) of the reference contour may be adjusted to increase the conformity of the reference contour to the surface contour of the bone. For example, where the patient's bone is a femur, the superior end of the scaled reference contour may be reduced or otherwise moved closer to the surface contour of the patient's femur in the region located superiorly to a cartilage demarcation line defined on the patient's femur. Conversely, where the patient's bone is a tibia, an inferior end of the scaled reference contour may be reduced or otherwise moved closer to the surface contour of the patient's tibia in the region located inferiorly to a cartilage demarcation line of the patient's tibia. As such, it should be appreciated that the scaled reference contour is initially enlarged to compensate for the thickness of the patient's cartilage on the patient's bone. Portions of the scaled reference contour are then reduced or otherwise moved back to original positions and/or toward the reference point in those areas where cartilage is lacking, reduced, or otherwise not present.

Once the reference contour has been scaled and adjusted in steps 46 and 48, the position of the cutting guide is defined in step 50. In particular, the position of the cutting guide is defined based on an angle defined between a mechanical axis of the patient's femur and a mechanical axis of the patient's tibia. The angle may be determined by establishing a line segment or ray originating from the proximal end of the patient's femur to the distal end of the patient's femur and defining a second line segment or ray extending from the patient's ankle through the proximal end of the patient's tibia. The angle defined by these two line segments/rays is equal to the angle defined between the mechanical axis of the patient's femur and tibia. The position of the bone cutting guide is then determined based on the angle between the mechanical axes of the patient's femur and tibia. It should be appreciated that the position of the cutting guide defines the position and orientation of the cutting plane of the customized patient-specific cutting block. Subsequently, in step 52, a negative contour of the customized patient-specific cutting block is defined based on the scaled and adjusted reference contour and the angle defined between the mechanical axis of the femur and tibia.

Referring back to FIG. 1, after the model of the customized patient-specific orthopaedic surgical instrument has been generated in process step 26, the model is validated in process step 28. The model may be validated by, for example, analyzing the rendered model while coupled to the three-dimensional model of the patient's anatomy to verify the correlation of cutting guides and planes, drilling guides and planned drill points, and/or the like. Additionally, the model may be validated by transmitting or otherwise providing the model generated in step 26 to the orthopaedic surgeon for review. For example, where the model is a three-dimensional rendered model, the model along with the three-dimensional images of the patient's relevant bone(s) may be transmitted to the surgeon for review. Where the model is a physical prototype, the model may be shipped to the orthopaedic surgeon for validation.

After the model has been validated in process step 28, the customized patient-specific orthopaedic surgical instrument is fabricated in process step 30. The customized patient-specific orthopaedic surgical instrument may be fabricated using any suitable fabrication device and method. Additionally, the customized patient-specific orthopaedic instrument may be formed from any suitable material such as a metallic material, a plastic material, or combination thereof depending on, for example, the intended use of the instrument. The fabricated customized patient-specific orthopaedic instrument is subsequently shipped or otherwise provided to the orthopaedic surgeon. The surgeon performs the orthopaedic surgical procedure in process step 32 using the customized patient-specific orthopaedic surgical instrument. As discussed above, because the orthopaedic surgeon does not need to determine the proper location of the orthopaedic surgical instrument intra-operatively, which typically requires some amount of estimation on part of the surgeon, the guesswork and/or intra-operative decision-making on part of the orthopaedic surgeon is reduced.

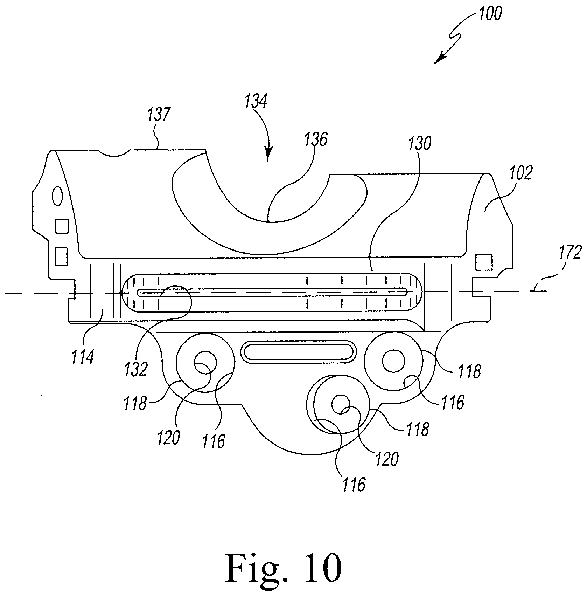

Referring now to FIGS. 10-12, in one construction, the customized patient-specific orthopaedic surgical instrument may be constructed as a tibial cutting block 100. The cutting block 100 is configured to be coupled to a tibia of a patient. The cutting block 100 includes a body 102 configured to be coupled to the anterior side of the patient's tibia and two arms or tabs 104, 106 which extend posteriorly away from the body 102. The tabs 104, 106 are configured to wrap around a proximal end of the tibia as discussed in more detail below. The cutting block 100 may be formed from any suitable material. For example, the cutting block 100 may be formed from a plastic or resin material. In one particular construction, the cutting block 100 is formed from Vero resin using a rapid prototype fabrication process. However, the cutting block 100 may be formed from other materials in other constructions. For example, in another particular construction, the cutting block 100 is formed from a polyimide thermoplastic resin, such as a Ultem resin, which is commercially available from Saudi Basic Industries Corporation Innovative Plastics of Riyhadh, Saudi Arabia.

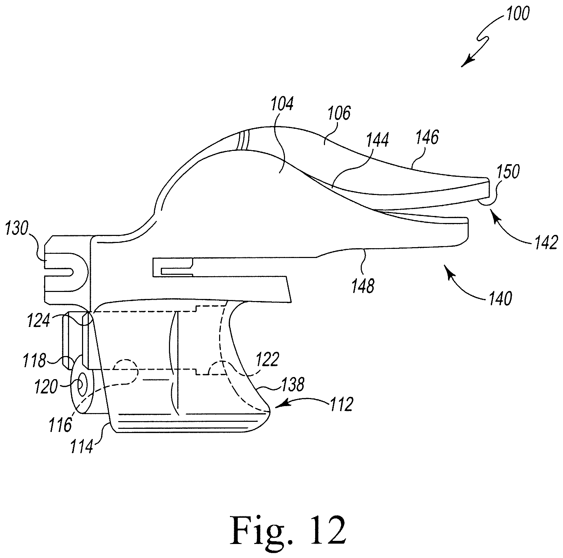

The body 102 includes a bone-contacting or bone-facing surface 112 and an outer surface 114 opposite the bone-facing surface 112. The outer surface 114 includes a number of guide holes or passageways 116 defined therethrough. A guide pin bushing 118 is received in each guide hole 116. The guide pin bushings 118 include an internal passageway 120 sized to receive a respective guide pin to secure the block 100 to the patient's tibia. As shown in FIG. 12, the guide passageways 116 extends from the outer surface 114 to the bone-facing surface 112 and is counterbored on the bone-facing surface 112. That is, the passageway 116 has an opening 122 on the bone-facing surface 112 having a diameter greater than the diameter of an opening 124 on the outer surface 114

The cutting guide 100 includes a cutting guide 130 secured to the body 102. In one particular construction, the cutting guide 130 is overmolded to the body 102. The cutting guide 130 includes a cutting guide slot 132. The cutting guide 130 may be formed from the same material as the body 102 or from a different material. In one particular construction, the cutting guide 130 is formed from a metallic material such as stainless steel. The body 102 also includes a window or opening 134 to allow a surgeon to visualize the positioning of the block 100 on the patient's tibia by viewing portions of the tibia through the opening 134. In the illustrative construction, the window 134 is embodied as a notch 136 defined on a superior end surface 137 of the body 102 of the cutting guide 100. However, in other constructions, the cutting block 100 may include windows or openings formed in the body 102 having other shapes and sizes.

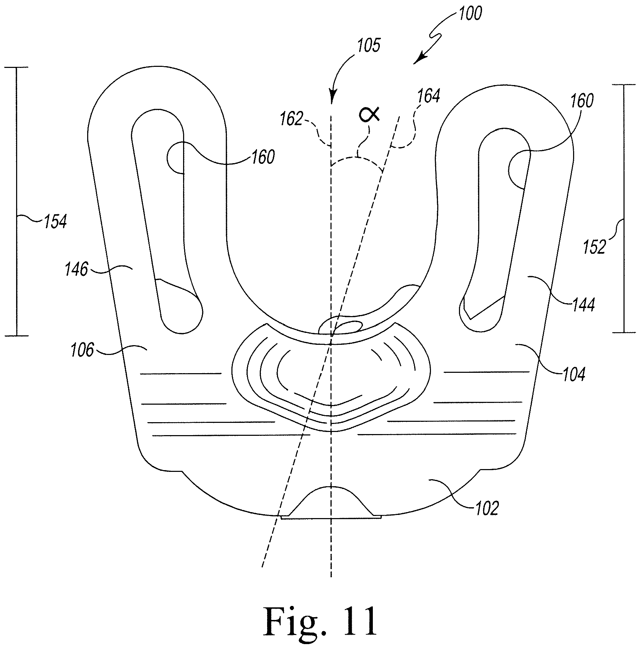

The bone-facing surface 112 of the body 102 includes a negative contour 138 configured to receive a portion of the anterior side of the patient's tibia having a corresponding contour and a portion of the medial side of the patient's tibia. The customized patient-specific negative contour 138 of the bone-contacting surface 112 allows the positioning of the cutting block 100 on the patient's tibia in a unique predetermined location and orientation. In the exemplary construction described herein, the negative contour 138 is selected such that cutting block 100 is configured to be coupled to the patient's tibia on an anterior-medial side, as opposed to solely on the anterior surface of the tibia. For example, as illustrated in FIG. 11, when the cutting block 100 is secured to a patient's tibia, an angle (α) is defined between a vertically-extending, bisecting plane 162 of the body 102 of the block 100 and a bisecting sagittal plane 164 of the patient's tibia (i.e., the A/P plane 164). The magnitude of the angle (α) may be selected based on, for example, the gender or age of the patient. In one particular construction, the angle (α) is in the range of about 10° to about 30°. In another particular construction, the angle is about 20°.

Such an offset from the A/P plane 164 facilitates use of the cutting block 100. In particular, conventional tibial cutting blocks are designed for attachment to the anterior surface of the proximal tibia. However, the patellar tendon and a significant amount of other soft tissues cover the proximal tibia in this region. Therefore, in order to secure a conventional tibial resection guide to the proximal tibia, the surgeon must create a long enough incision to retract the patellar tendon and soft tissue layers. However, the region of the proximal tibia slightly medial to the patellar tendon has relatively little soft tissue overlying the bone surface. As such, the customized patient-specific negative contour 138 of the bone-contacting surface 112 allows the positioning of the cutting block 100 to be secured to this medial-anterior region of the proximal tibia in order to minimize trauma to the patellar tendon and soft tissues. Moreover, the medial-anterior region of the proximal tibia includes a number of anatomical features that make for "high confidence" placement of the cutting block 100.

As shown in FIG. 13, in such a configuration, the cutting slot 132 is likewise oriented in the offset position to guide a bone saw directed in the anterior-posterior plane. In other words, when viewed superiorly as in FIG. 13, the longitudinal axis 172 of the cutting slot 132 is perpendicular to the bisecting plane 162 of the cutting block's body 102.

The tabs 104, 106 include a bone-contacting or bone-facing surface 140, 142, respectively, and an outer surface 144, 146, respectively, opposite the bone-facing surface 140, 142. The bone-facing surface 140 of the tab 104 includes a negative contour 148 configured to receive a portion of the proximal side of the patient's tibia having a respective corresponding contour. Similarly, the bone-facing surface 142 of the tab 106 includes a negative contour 150 configured to receive a portion of the proximal side of the patient's tibia having a respective corresponding contour.

As discussed above, the arms or tabs 104, 106 extend posteriorly from the body 102 to define a U-shaped opening 105 therebetween. The tabs 104, 106 may extend from the body 102 the same distance or a different distance. For example, as shown in FIG. 11, the tab 104 extends from the body 102 a distance 152 and the tab 106 extends from the body 102 by a distance 154, which is greater than the distance 152.

In some constructions, the negative contours 138, 148, 150 of the bone-contacting surfaces 112, 140, 142 of the cutting block 1400 may or may not match the corresponding contour surface of the patient's bone. That is, as discussed above, the negative contours 138, 148, 150 may be scaled or otherwise resized (e.g., enlarged) to compensate for the patient's cartilage or lack thereof.

In use, the tibial cutting block 100 is coupled to the proximal end of the patient's tibia. Again, because the bone-contacting surfaces 112, 140, 142 of the cutting block 100 include the negative contours 138, 148, 150 the block 100 may be coupled to the patient's tibia in a pre-planned, unique position. When so coupled, the tabs 104, 106 wrap around the proximal end of the patient's tibia and the lips 108, 110 of the tabs 104, 106 wrap around the posterior side of the patient's tibia. Additionally, when the block 100 is coupled to the patient's tibia, a portion of the anterior side of the tibia is received in the negative contour 138 of the body 102 and a portion of the proximal side of the patient's tibia is received in the negative contours 148, 150 of the tabs 104, 106.

Referring now to FIG. 14, there is shown an embodiment of the tibial cutting block 100. The tibial cutting block 100 of FIG. 14 is substantially the same as the tibial cutting block 100 of FIGS. 10-13 except for the orientation of the cutting slot 132. In particular, like the cutting block 100 of FIGS. 10-13, the customized patient-specific negative contour 138 of the bone-contacting surface 112 of the cutting block 100 if FIG. 14 is configured to be coupled to the patient's tibia on an anterior-medial side, as opposed to solely on the anterior surface of the tibia. In particular, as shown in FIG. 14, like the cutting block 100 of FIGS. 10-13, when the cutting block 100 of FIG. 14 is secured to a patient's tibia, an angle (α) is defined between the vertically-extending, bisecting plane 162 of the body 102 of the block 100 and the bisecting sagittal plane 164 of the patient's tibia (i.e., the A/P plane 164). The magnitude of the angle (α) may be selected based on, for example, the gender or age of the patient. In one particular embodiment, the angle (α) is in the range of about 10° to about 30°. In another particular embodiment, the angle is about 20°.

However, unlike the cutting block 100 of FIGS. 10-13, the cutting slot 132 of the cutting block 100 of FIG. 14 is oriented in the position to guide a bone saw directed in the anterior-posterior plane. In other words, when viewed superiorly as in FIG. 14, the longitudinal axis 172 of the cutting slot 132 is perpendicular to the bisecting sagittal plane 164 of the patient's tibia (i.e., the A/P plane 164). Such a configuration allows the cutting block 100 to be secured to the "high confidence" anatomical features associated with the medial-anterior region of the proximal tibia, while also allowing for advancement of the bone saw blade along the A/P plane 164. It is to be understood that the drawings and description are illustrative only and that the invention is defined and limited only by the scope of the appended claims.

## Claims

1. A customized patient-specific tibial cutting block (100), comprising:
a body (100) having a bone-facing surface (112) that has a customized patient-specific negative contour (138) configured to receive (i) a portion of an anterior side of a patient's tibia that has a corresponding positive contour, and (ii) a portion of a medial side of the patient's tibia that has a corresponding positive contour such that, when viewed superiorly, an angle greater than zero is defined between a vertically-extending, bisecting plane (162) of the body and a bisecting saggital plane (164) of the patient's tibia when the portions of the patient's tibia are received in the customized patient-specific negative contour of the body, and
at least one tab (104, 106) extending posteriorly from the body, the at least one tab having a bone-facing surface (140, 142) having a customized patient-specific negative contour (148, 150) configured to receive a portion of the proximal side of the patient's tibia that has a corresponding positive contour and further comprising a cutting guide (132) coupled to the body, in which the cutting guide has a cutting slot defined therein, the cutting slot having a longitudinal axis, and **characterised in that** the longitudinal axis (172) of the cutting slot defines a perpendicular angle with the bisecting sagittal plane of the patient's tibia when the portions of the patient's tibia are received in the customized patient-specific negative contour of the body.

2. The customized patient-specific tibial cutting block (100) of claim 1, in which:
the at least one tab (104, 106) comprises a first tab (104) extending posteriorly from the body (102) and a second tab (106) extending posteriorly from the body,
each of the first tab and the second tab has a bone-facing surface having a customized patient-specific negative contour (148, 150) configured to receive a respective portion of the proximal end of the patient's tibia that has a corresponding positive contour, and
the first tab and the second tab define an opening (105) therebetween.

3. The customized patient-specific tibial cutting block (100) of claim 1, in which the cutting guide (132) is formed from a material different from the body (102).

4. The customized patient-specific tibial cutting block (100) of claim 3 in which the cutting guide is formed from a metallic material and is over-moulded to the body of the customized patient-specific tibial cutting block.

5. The customized patient-specific tibial cutting block (100) of claim 1, in which the angle defined between the vertically-extending, bisecting plane (162) of the body and the bisecting sagittal plane of the patient's tibia (164) is between ten degrees and thirty degrees, optionally about twenty degrees.

## Patentansprüche

1. Maßgeschneiderter patientenspezifischer Tibia- bzw. Schienbeinschneidblock (100), umfassend:
einen Körper (100) mit einer knochenzugewandten Fläche bzw. Oberfläche (112), die eine maßgeschneiderte patientenspezifische negative Kontur (138) aufweist, die konfiguriert ist, (i) einen Abschnitt einer anterioren Seite des Tibia bzw. Schienbeins eines Patienten mit einer entsprechenden bzw. korrespondieren positiven Kontur und (ii) einen Abschnitt einer medialen Seite des Tibia bzw. Schienbeins eines Patienten mit einer entsprechenden bzw. korrespondieren positiven Kontur aufzunehmen, so dass bei Betrachtung von oben ein Winkel größer Null zwischen einer sich vertikal erstreckenden, halbierenden Ebene (162) des Körpers und einer halbierenden Saggitalebene (164) des Schienbeins des Patienten definiert ist, wenn die Abschnitte des Schienbeins des Patienten in der maßgeschneiderten patientenspezifischen negativen Kontur des Körpers aufgenommen sind, und
zumindest eine Lasche (104, 106), die sich posterior von dem Körper erstreckt, wobei die zumindest eine Lasche eine knochenzugewandte Fläche bzw. Oberfläche (140, 142) mit einer maßgeschneiderten patientenspezifischen negativen Kontur (148, 150) aufweist, die konfiguriert ist, einen Abschnitt der proximalen Seite des Schienbeins des Patienten mit einer entsprechenden bzw. korrespondierenden positiven Kontur aufzunehmen, und ferner umfassend eine Schneidführung (132), die mit dem Körper gekoppelt ist, wobei die Schneidführung einen darin definierten Schneidschlitz aufweist, wobei der Schneidschlitz eine Längsachse aufweist,
**dadurch gekennzeichnet, dass** die Längsachse (172) des Schneidschlitzes einen senkrechten Winkel mit der halbierenden Saggitalebene des Schienbeins des Patienten definiert, wenn die Abschnitte des Schienbeins des Patienten in der maßgeschneiderten patientenspezifischen negativen Kontur des Körpers aufgenommen sind.

2. Maßgeschneiderter patientenspezifischer Tibiaschneidblock (100) nach Anspruch 1, wobei:
die zumindest eine Lasche (104, 106) eine erste Lasche (104), die sich posterior von dem Körper (102) erstreckt, und eine zweite Lasche (106) umfasst, die sich posterior von dem Körper erstreckt,
jede der ersten Lasche und der zweiten Lasche eine knochenzugewandte Fläche bzw. Oberfläche mit einer maßgeschneiderten patientenspezifischen negativen Kontur (148, 150) aufweist, die konfiguriert ist, einen jeweiligen Abschnitt des proximalen Endes des Schienbeins des Patienten aufzunehmen, der eine entsprechende bzw. korrespondierende positive Kontur aufweist, und die erste Lasche und die zweite Lasche eine Öffnung (105) dazwischen bzw. zwischen sich definieren.

3. Maßgeschneiderter patientenspezifischer Tibiaschneidblock (100) nach Anspruch 1, wobei die Schneidführung (132) aus einem Material besteht, das sich von dem Körper (102) unterscheidet.

4. Maßgeschneiderter patientenspezifischer Tibiaschneidblock (100) nach Anspruch 3, wobei die Schneidführung aus einem metallischen Material besteht und auf den Körper des maßgeschneiderten patientenspezifischen Tibiaschneidblocks aufgeformt ist.

5. Maßgeschneiderter patientenspezifischer Tibiaschneidblock (100) nach Anspruch 1, wobei der Winkel, der zwischen der sich vertikal erstreckenden, halbierenden Ebene (162) des Körpers und der halbierenden Sagittalebene des Schienbeins (164) des Patienten definiert ist, zwischen zehn Grad und dreißig Grad, optional etwa zwanzig Grad beträgt.

## Revendications

1. Bloc de coupe tibiale spécifique au patient personnalisé (100), comprenant :
un corps (100) qui comporte une surface faisant face à l'os (112) qui présente un profil négatif spécifique au patient personnalisé (138) qui est configuré de manière à ce qu'il reçoive (i) une partie d'un côté antérieur d'un tibia de patient qui présente un profil positif correspondant et (ii) une partie d'un côté médial du tibia de patient qui présente un profil positif correspondant de telle sorte que, tel que vu de dessus, un angle supérieur à zéro soit défini entre un plan bissecteur s'étendant verticalement (162) du corps et un plan sagittal bissecteur (164) du tibia de patient lorsque les parties du tibia de patient sont reçues dans le profil négatif spécifique au patient personnalisé du corps ; et
au moins une patte (104, 106) qui s'étend de façon postérieure à partir du corps, l'au moins une patte comportant une surface faisant face à l'os (140, 142) qui présente un profil négatif spécifique au patient personnalisé (148, 150) qui est configuré de manière à ce qu'il reçoive une partie du côté proximal du tibia de patient qui présente un profil positif correspondant et comprenant en outre un guide de coupe (132) qui est couplé au corps, dans lequel le guide de coupe comporte une fente de coupe qui est définie en son sein, la fente de coupe présentant un axe longitudinal ; et
**caractérisé en ce que** l'axe longitudinal (172) de la fente de coupe définit un angle droit avec le plan sagittal bissecteur du tibia de patient lorsque les parties du tibia de patient sont reçues dans le profil négatif spécifique au patient personnalisé du corps.

2. Bloc de coupe tibiale spécifique au patient personnalisé (100) selon la revendication 1, dans lequel :
l'au moins une patte (104, 106) comprend une première patte (104) qui s'étend de façon postérieure à partir du corps (102) et une seconde patte (106) qui s'étend de façon postérieure à partir du corps ;
chaque patte prise parmi la première patte et la seconde patte comporte une surface faisant face à l'os qui présente un profil négatif spécifique au patient personnalisé (148, 150) qui est configuré de manière à ce qu'il reçoive une partie respective de l'extrémité proximale du tibia de patient qui présente un profil positif correspondant ; et
la première patte et la seconde patte définissent une ouverture (105) entre elles.

3. Bloc de coupe tibiale spécifique au patient personnalisé (100) selon la revendication 1, dans lequel le guide de coupe (132) est formé à partir d'un matériau qui est différent de celui du corps (102).

4. Bloc de coupe tibiale spécifique au patient personnalisé (100) selon la revendication 3, dans lequel ledit guide de coupe est formé à partir d'un matériau métallique et est surmoulé sur le corps du bloc de coupe tibiale spécifique au patient personnalisé.

5. Bloc de coupe tibiale spécifique au patient personnalisé (100) selon la revendication 1, dans lequel l'angle qui est défini entre le plan bissecteur s'étendant verticalement (162) du corps et le plan sagittal bissecteur du tibia de patient (164) s'inscrit entre dix degrés et trente degrés et en option, il vaut environ 20 degrés.
